(19) Europäisches Patentamt
European Patent Office
Office européen des brevets

(11) Veröffentlichungsnummer: **0 308 782 A2**

(12) **EUROPÄISCHE PATENTANMELDUNG**

(21) Anmeldenummer: **88114933.0**

(22) Anmeldetag: **13.09.88**

(51) Int. Cl.[4]: **A61K 31/41 , A61K 31/415 , C07D 403/06 , C07D 249/08**

(30) Priorität: **25.09.87 DE 3732384**

(43) Veröffentlichungstag der Anmeldung: **29.03.89 Patentblatt 89/13**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Holmwood, Graham, Dr.**
**Krutscheider Weg 105**
**D-5600 Wuppertal 11(DE)**
Erfinder: **Krämer, Wolfgang, Dr.**
**Rosenkranz 25**
**D-5093 Burscheid 2(DE)**
Erfinder: **Regel, Erik, Dipl.-Ing.**
**Untere Bergerheide 26**
**D-5600 Wuppertal 1(DE)**
Erfinder: **Elbe, Hans-Ludwig, Dr.**
**Dasnöckel 59**
**D-5600 Wuppertal 11(DE)**
Erfinder: **Büchel, Karl Heinz, Prof. Dr.**
**Dabringhausener Strasse 42**
**D-5093 Burscheid(DE)**
Erfinder: **Plempel, Manfred, Dr.**
**Zwengenberger Strasse 3c**
**D-5657 Haan(DE)**

(54) **Antimykotische Mittel.**

(57) Die vorliegende Erfindung betrifft neue Bisazolyl-hydroxyalkyl-Derivate sowie deren pharmakologisch verträgliche Säureadditions-Salze zur Bekämpfung von Krankheiten, insbesondere Mykosen.

EP 0 308 782 A2

## Antimykotische Mittel

Die vorliegende Erfindung betrifft neue Bisazolyl-hydroxyalkyl-Derivate und deren pharmakologisch verträgliche Säureadditions-Salze zur Behandlung von Krankheiten, insbesondere Mykosen.

Es ist bereits allgemein bekannt geworden, daß bestimmte Diazolyl-Derivate, wie beispielsweise 1,3-Di-(1,2,4-triazol-1-yl)-2-(4-chlorphenyl)- bzw. -phenyl-2-propanol, antimykotische Eigenschaften aufweisen (vergleiche EP-OS 0 044 605). Die Wirkung dieser Stoffe ist jedoch nicht immer in allen Indikationsbereichen voll zufriedenstellend.

Es wurde gefunden, daß die neuen Bisazolyl-hydroxyalkyl-Derivate der allgemeinen Formel (I),

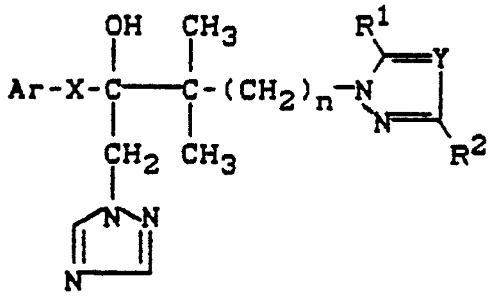

(I)

in welcher

Ar für gegebenenfalls substituiertes Aryl steht,

X für eine Gruppierung - $CH_2CH_2$-, -CH=CH-, -$OCH_2$-, oder -$SCH_2$- steht, wobei das Sauerstoff- oder Schwefelatom mit dem Arylrest direkt verbunden ist,

Y für ein Stickstoffatom oder die Gruppierung $CR^3$ steht,

$R^1$, $R^2$ und $R^3$ für Wasserstoff, Halogen oder Methyl stehen und

n für die Zahlen 0, 1 oder 2 steht,

sowie deren Säureadditions-Salze gute antimikrobielle, insbesondere antimykotische Eigenschaften aufweisen.

Die neuen Bisazolyl-hydroxyalkyl-Derivate der Formel (I) besitzen ein asymmetrisches Kohlenstoffatom und können deshalb in den beiden optischen Isomerenformen anfallen. Die Erfindung betrifft sowohl die Racemate als auch die einzelnen Isomeren und deren Gemische.

Außerdem können die erfindungsgemäßen Verbindungen der Formel (I) für X = -CH=CH- in den geometrischen Isomerenformen cis und trans vorkommen. Die Erfindung betrifft sowohl die einzelnen Isomeren als auch deren Gemische.

Die erfindungsgemäßen Bisazolyl-hydroxyalkyl-Derivate sind durch die Formel (I) allgemein definiert.

Bevorzugt sind diejenigen Verbindungen der Formel (I), in denen

Ar für gegebenenfalls einfach bis dreifach, gleich oder verschieden substituiertes Phenyl steht, wobei als Substituenten genannt seien: Halogen, Alkyl mit 1 bis 4 Kohlenstoffatomen, Alkoxy und Alkylthio mit jeweils 1 bis 2 Kohlenstoffatomen, Nitro, Halogenalkyl, Halogenalkoxy und Halogenalkylthio mit jeweils 1 bis 2 Kohlenstoffatomen und 1 bis 5 gleichen oder verschiedenen Halogenatomen, wie vorzugsweise Fluor- und Chloratomen, Alkoximinoalkyl mit 1 bis 4 Kohlenstoffatomen im Alkoxyteil und 1 bis 2 Kohlenstoffatomen im Alkylteil sowie jeweils gegebenenfalls durch Halogen und Alkyl mit 1 bis 2 Kohlenstoffatomen substituiertes Phenyl, Phenoxy, Benzyl und Benzyloxy;

Ar außerdem für gegebenenfalls einfach bis dreifach, gleich oder verschieden substituiertes Naphthyl steht, wobei als Substituenten gennant seien: Halogen, Alkyl und Alkoxy mit jeweils 1 bis 2 Kohlenstoffatomen sowie Halogenalkoxy mit 1 bis 2 Kohlenstoffatomen und 1 bis 5 gleichen oder verschiedenen Halogenatomen, wie vorzugsweise Fluor-und Chloratomen;

X für Gruppierungen -$CH_2CH_2$-, -$OCH_2$-, -$SCH_2$- oder -CH=CH- steht;

Y für ein Stickstoffatom oder die Gruppierung $CR^3$ steht;

$R^1$, $R^2$ und $R^3$ für Wasserstoff, Chlor, Brom oder Methyl stehen und

n für die Zahlen 0, 1 oder 2 steht.

Besonders bevorzugt sind diejenigen Verbindungen der Formel (I), in denen

Ar für gegebenenfalls einfach bis dreifach, gleich oder verschieden substituiertes Phenyl steht, wobei als

Substituenten genannt seien: Fluor, Chlor, Brom, Methyl, Methoxy, Methylthio, Nitro, Trifluormethyl, Trifluormethoxy, Trifluormethylthio, Methoximinomethyl, Methoximinoethyl, Ethoximinomethyl sowie jeweils gegebenenfalls durch Fluor, Chlor und Methyl substituiertes Phenyl, Phenoxy, Benzyl und Benzyloxy;
Ar außerdem für Naphthyl steht;
X für die Gruppierungen $-CH_2CH_2-$, $-OCH_2-$, $-SCH_2-$ oder $-CH=CH-$ steht;
Y für ein Stickstoffatom oder die Gruppierung $CR^3$ steht;
$R^1$, $R^2$ und $R^3$ für Wasserstoff, Chlor oder Methyl stehen und
n für die Zahlen 0, 1 oder 2 steht.

Bevorzugte erfindungsgemäße Verbindungen sind auch pharmakologisch veträgliche Additionsprodukte aus Säuren und denjenigen Bisazolyl-hydroxyalkyl-Derivaten der Formel (I), in denen Ar, X, Y, $R^1$, $R^2$, $R^3$ und n diejenigen Bedeutungen haben, die bereits im Zusammenhang mit der Beschreibung der erfindungsgemäßen Stoffe für diese Substituenten bzw. diesen Index als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Die erfindungsgemäß zu verwendenden Bisazolyl-hydroxyalkyl-Derivate sowie ihre Säureadditions-Salze können in allgemein bekannter Art und Weise erhalten werden, indem man Oxirane der allgemeinen Formel (II)

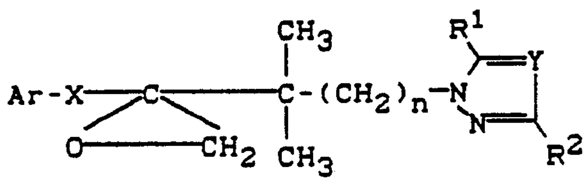

(II)

in welcher
Ar, X, Y, $R^1$, $R^2$ und n die oben angegebene Bedeutung haben,
mit 1,2,4-Triazol der Formel (III)

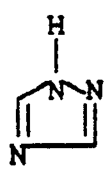

(III)

in Gegenwart eines Verdünnungsmittels, wie beispielsweise Alkohole oder Dimethylformamid und gegebenenfalls in Gegenwart eines Säurebindemittels, wie beispielsweise Alkalihydroxide sowie gegebenenfalls in Gegenwart eines Katalysators, wie beispielsweise $\alpha,\alpha'$-Azo-isobutyronitril umsetzt und gegebenenfalls anschließend an die so erhaltenen Verbindungen der Formel (I) in üblicher Art und Weise eine Säure addiert.

Die Oxirane der Formel (II) sind noch nicht bekannt. Sie lassen sich herstellen, indem man

a) in einer <u>ersten Stufe</u> Ketone der Formel (IVa)

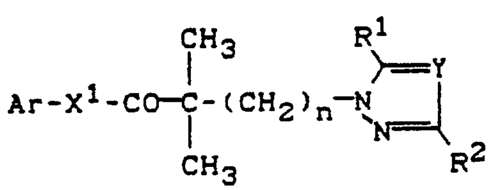

(IVa)

in welcher
Ar, Y, $R^1$, $R^2$ und n die oben angegebenem Bedeutung haben und

$X^1$ für die Gruppierungen $-CH_2CH_2-$, $-OCH_2-$ und $-SCH_2-$ steht,
mit Methyl-triphenyl-phosphoniumbromid der Formel (V)

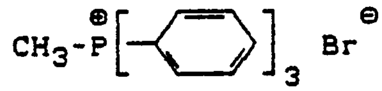

(V)

in Gegenwart einer Base und in Gegenwart eines Verdünnungsmittels umsetzt und dann in einer zweiten Stufe die so erhaltenen Verbindungen der Formel (VI)

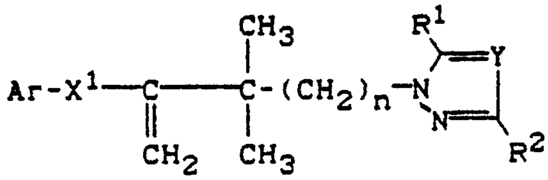

(VI)

in welcher
Ar, $X^1$, Y, $R^1$, $R^2$ und n die oben angegebene Bedeutung haben,
mit Persäure in Gegenwart eines Verdünnungsmittels umsetzt;
oder

(b) Ketone der Formel (IVb)

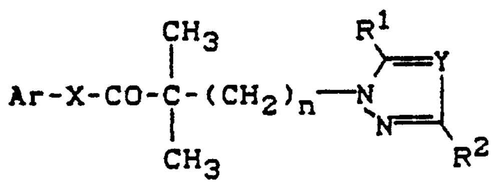

(IVb)

in welcher
Ar, X, Y, $R^1$, $R^2$ und n die oben angegebene Bedeutung haben,
entweder
α) mit Dimethyloxosulfonium-methylid der Formel

$$\overset{\delta\oplus}{(CH_3)_2SO}\ \overset{\delta\ominus}{CH_2} \qquad (VII)$$

oder
β) mit Dimethylsulfonium-methylid der Formel

$$\overset{\delta\oplus}{(CH_3)_2S}\ \overset{\delta\ominus}{CH_2} \qquad (VIII)$$

in Gegenwart eines Verdünnungsmittels umsetzt.

Die bei der Herstellung der Oxirane der Formel (II) als Ausgangsstoffe benötigten Ketone der Formel (IVa) bzw. (IVb) sind noch nicht bekannt; sie lassen sich nach im Prinzip bekannten Verfahren herstellen (vgl. z.B. EP-OS 0 084 834). So erhält man Ketone der Formel (IVc)

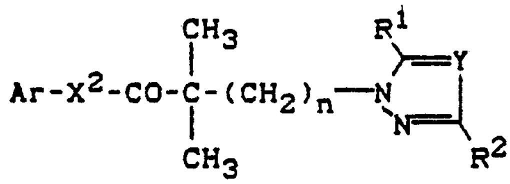 (IVc)

in welcher
Ar, Y, $R^1$, $R^2$ und n die oben angegebene Bedeutung haben und
$X^2$ für die Gruppierungen $-OCH_2-$ und $-SCH_2-$stehen,
indem man Halogenketone der Formel (IX)

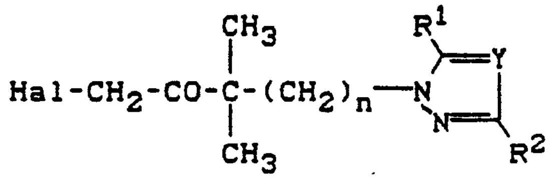 (IX)

in welcher
Y, $R^1$, $R^2$ und n die oben angegebene Bedeutung haben und
Hal für Chlor oder Brom steht,
mit Verbindungen der Formel (X)

Ar-Z-H (X)

in welcher
Ar die oben angegebene Bedeutung hat und
Z für Sauerstoff oder Schwefel steht,
in Gegenwart einer Base, wie z.B. Kaliumcarbonat oder Triethylamin und in Gegenwart eines Verdünnungsmittels, wie z.B. Aceton oder Acetonitril, bei Temperaturen zwischen 20 °C und 100 °C umsetzt.

Weiterhin erhält man Ketone der Formel (IVd)

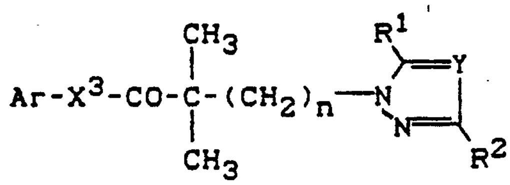 (IVd)

in welcher
Ar, Y, $R^1$, $R^2$ und n die oben angegebene Bedeutung haben und
$X^3$ für die Gruppierungen $-CH_2CH_2-$ und $-CH=CH-$ stehen,
in dem man Verbindungen der Formel (XI)

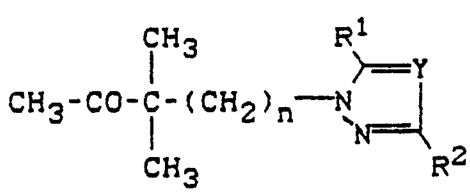 (XI)

in welcher
Y, $R^1$, $R^2$ und n die oben angegebene Bedeutung haben
mit Aldehyden der Formel (XII)

Ar-CHO (XII)

in welcher
Ar die oben angegebene Bedeutung hat,
in Gegenwart eines Verdünnungsmittels, wie Ethanol und einer Base wie Natriumhydroxid, bei Temperaturen zwischen 0 und 60 °C umsetzt und gegebenenfalls die entstehenden Verbindungen der Formel (IVe)

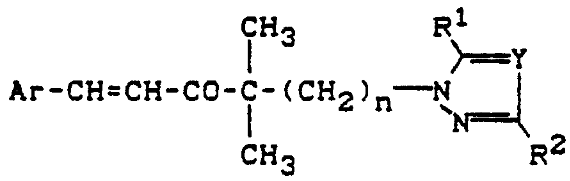 (IVe)

in welcher
Ar, $R^1$, $R^2$, Y und n die oben angegebene Bedeutung haben,
mit Wasserstoff in Gegenwart eines Katalysators, wie z.B. Raney-Nickel, in Gegenwart eines Verdünnungsmittels, wie z.B. Methanol, Toluol oder Tetrahydrofuran, bei Temperaturen zwischen 40 und 180 °C hydriert (vgl. auch die Herstellungsbeispiele).
In manchen Fällen erweist es sich als vorteilhaft, neben der -CH = CH-Doppelbindung gleichzeitig die Keto-Gruppe mitzuhydrieren und diese anschließend in üblicher Art und Weise, wie z.B. mit Chromsäure/Eisessig, wieder zu oxidieren (vgl. auch die Herstellungsbeispiele).

Die Halogenketone der Formel (IX) werden in bekannter Art und Weise durch übliche Halogenierung der Verbindungen der Formel (XI) erhalten.

Die Verbindungen der Formel (XI) werden in bekannter Art und Weise erhalten, indem man Verbindungen der Formel (XIII)

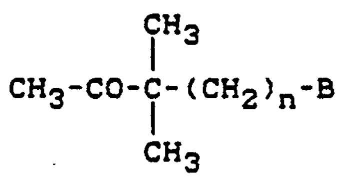 (XIII)

in welcher
B für eine übliche Abgangsgruppe, wie Chlor, Brom, Methylsulfonyloxy oder p-Methylphenylsulfonyloxy steht und
n die oben angegebene Bedeutung hat
mit Azolen der Formel (XIV)

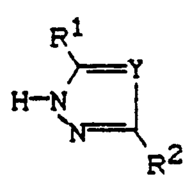 (XIV)

in welcher
Y, $R^1$ und $R^2$ die oben angegebene Bedeutung haben,
in der Schmelze oder gegebenenfalls in Gegenwart eines Lösungsmittels, wie z.B. Aceton und in Gegenwart eines Säurebindemittels, wie z.B. Kaliumcarbonat, bei Temperaturen zwischen 60 und 120 °C umsetzt. Die Azole der Formel (XIV) können aber auch in Form ihrer Alkalisalze, die in üblicher Weise in-situ hergestellt werden, eingesetzt werden.

Sowohl die Verbindungen der Formel (X) und (XIII) als auch die Aldehyde der Formel (XII) und die Azole der Formel (XIV) sind bekannt oder lassen sich nach bekannten Verfahren herstellen.

Das bei der Herstellung der Oxirane der Formel (II) nach dem Verfahren (a) weiterhin als Ausgangsmaterial benötigte Methyl-triphenyl-phosphonium-bromid der Formel (V) ist bekannt.

Die bei der Herstellung der Oxirane der Formel (II) nach dem obigen Verfahren (a) in der zweiten Stufe als Ausgangssubstanzen benötigten Verbindungen der Formel (VI) sind bisher noch nicht bekannt.

Bei dem Verfahren (a) zur Herstellung der Oxirane der Formel (II) arbeitet man in der ersten Stufe in Gegenwart einer Base. Dabei kommen als Basen alle üblicherweise für Wittig-Reaktionen dieser Art verwendbaren Basen in Betracht. Vorzugsweise verwendbar ist Kalium-tert.-butylat.

Als Verdünnungsmittel kommen bei der Durchführung der ersten Stufe des obigen Verfahrens (a) zur Herstellung der Oxirane der Formel (II) alle für derartige Umsetzungen üblichen organischen Solventien infrage. Vorzugsweise verwendbar sind aromatische Kohlenwasserstoffe, wie Benzol, Toluol und Xylol.

Bei der Durchführung der zweiten Stufe des obigen Verfahrens (a) zur Herstellung der Oxirane der Formel (II) kommen als Reagenzien zur Epoxidierung alle üblichen Persäuren in Betracht. Vorzugsweise verwendbar sind meta-Chlorperbenzoesäure und Peressigsäure. Ferner ist es auch möglich, ein Gemisch aus Essigsäure und Wasserstoffperoxid einzusetzen.

Als Verdünnungsmittel kommen bei der Durchführung der zweiten Stufe des obigen Verfahrens (a) zur Herstellung der Oxirane der Formel (II) alle für derartige Epoxidierungen üblichen Solventien infrage. Vorzugsweise verwendbar sind Dichlormethan, Chloroform, Toluol, Dichlorbenzol, Essigsäure und andere inerte Solventien.

Die Reaktionstemperaturen können bei der Durchführung des Verfahrens (a) zur Herstellung der Oxirane der Formel (II) innerhalb eines bestimmten Bereiches variiert werden. Im allgemeinen arbeitet man in der ersten Stufe bei Temperaturen zwischen 50 °C und 140 °C, vorzugsweise zwischen 80 °C und 120 °C. In der zweiten Stufe arbeitet man im allgemeinen zwischen 10 °C und 60 °C, vorzugsweise zwischen 20 °C und 50 °C.

Bei der Durchführung des Verfahrens (a) zur Herstellung der Oxirane der Formel (II) geht man im allgemeinen so vor, daß man in der ersten Stufe auf 1 Mol an Keton der Formel (IVa) zwischen 1 und 3 Mol Methyl-triphenylphosphonium-bromid der Formel (V) sowie zwischen 1 und 3 Mol Base einsetzt. In der zweiten Stufe setzt man auf 1 Mol an einer Verbindung der Formel (VI) jeweils zwischen 1 und 2 Mol an Persäure ein. Die Aufarbeitung erfolgt jeweils nach üblichen Methoden.

Das bei dem Verfahren (b) als Reaktionskomponente benötigte Dimethyloxosulfoniummethylid der Formel (VII) ist bekannt (vgl. J. Amer. Chem. Soc. 87, 1363-1364 (1965)). Es wird bei der obigen Umsetzung in frisch zubereitetem Zustand verarbeitet, indem man es in situ durch Umsetzung von Trimethyloxo-sulfonium-iodid mit Natriumhydrid oder Natriumamid, insbesondere mit Kalium-tert.-butylat oder Natriummethylat, in Gegenwart eines Verdünnungsmittels erzeugt.

Das bei dem Verfahren (b) außerdem als Reaktionskomponente in Betracht kommende Dimethylsulfonium-methylid der Formel (VIII) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsulfonium-methylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (b) inerte organische Solventien infrage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei dem Verfahren (b) in einem größeren Bereich variiert werden. Im allgemeinen arbeitet man bei Temperaturen zwischen 0 und 100 °C, vorzugsweise zwischen 10 und 60 °C.

Bei der Durchführung des Verfahrens (b) setzt man auf 1 Mol an Keton der Formel (IVb), vorzugsweise 1 bis 3 Mol an Dimethyloxosulfonium-methylid der Formel (VII) bzw. an Dimethylsulfonium-methylid der Formel (VIII) ein. Die Isolierung der Oxirane erfolgt nach üblichen Methoden.

Die Oxirane der Formel (II) können bei dem erfindungsgemäßen Verfahren gegebenenfalls ohne Isolierung direkt weiter umgesetzt werden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegenbenenfalls durch Waschen mit einem inerten organischen Lösungsmittel gereinigt werden.

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum,

Aspergillus-Arten, wie Aspergillus niger und Anspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelatine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Träger stoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotoner

Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungs gemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele:

Beispiel 1

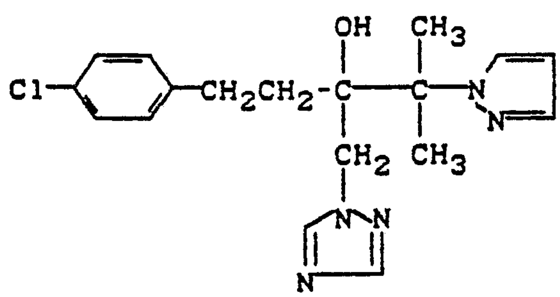

(I-1)

Zu 26 g (0.08 Mol) rohem 2-(4-Chlorphenylethyl)-2-(pyrazol-1-yl-prop-2-yl)-oxiran in 150 ml n-Butanol werden 11 g (0,16 Mol) 1,2,4-Triazol und 0,9 g (0,016 Mol) gepulvertes Kaliumhydroxid gegeben und die Mischung 10 Stunden bei 120 °C gerührt. Danach wird das Butanol im Wasserstrahlvakuum abdestilliert,

der Rückstand wird in 200 ml Methylenchlorid aufgenommen, die organische Phase wird 3 mal mit je 150 ml Wasser gewaschen, über Natriumsulfat getrocknet und das Lösungsmittel im Wasserstrahlvakuum abdestilliert. Man erhält 24 g oelige Substanz die durch Säulenchromatographie mit Methylenchlorid an Kieselgel 60 Merck gereinigt wird. Nach Umkristallisation der gewonnenen Fraktionen erhält man 4,6 g (16 % der Theorie) 5-(4-Chlorphenyl)-2-methyl-2-(pyrazol-1-yl)-3-(1,2,4-triazol-methyl)-pentan-3-ol vom Schmelzpunkt 118 °C.
Weitere 5 g können durch wiederholte Säulenchromatographie erhalten werden.

Herstellung von Ausgangsprodukten

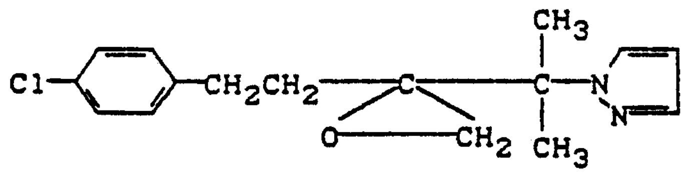

(II-1)

**(Variante b)**

17,6 g (0.08 Mol) Trimethylsulfoniumiodid werden in 50 ml Dimethylsulfoxid suspendiert, 9 g (0.08 Mol) Kalium-tert-butylat werden innerhalb von 30 Minuten portionsweise eingetragen und 1 Stunde bei Raumtemperatur nachgerührt. Bei 5 °C wird innerhalb einer Stunde eine Lösung von 22,1 g (0,08 Mol) 5-(4-Chlorphenyl)-2-methyl-2-(pyrazol-1-yl)-pentan-3-on in 20 ml Dimethylsulfoxid zugetropft und nach 22 Stunden Rühren bei Raumtemperatur die Mischung in 1000 ml Wasser eingerührt. Man extrahiert 3 mal mit 150 ml Methylenchlorid, trocknet die organische Phase und destilliert das Lösungsmittel im Wasserstrahlvakuum ab. Das erhaltene Oxiran wird ohne weitere Reinigung eingesetzt.

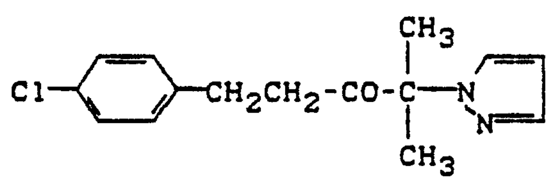

(IV-1)

23,5 g (0,085 Mol) 5-(4-Chlorphenyl)-2-methyl-2-(pyrazol-1-yl)-pentan-3-ol werden in 60 ml Eisessig gelöst, bei 5 °C innerhalb einer Stunde eine Lösung von 6,4 g (0,064 Mol) Chromsäure in 5 ml Wasser und 20 ml Eisessig zugetropft und weitere 25 Stunden bei Raumtemperatur nachgerührt. Man rührt die Lösung in 2000 ml Wasser, extrahiert 5 mal mit je 200 ml Methylenchlorid und destilliert das Lösungsmittel ab.
Man erhält 22,1 g (94 % der Theorie) 5-(4-Chlorphenyl)-2-methyl-2-(pyrazol-1-yl)-pentan-3-on vom Brechungsindex $n_D^{23}$ = 1,5278.

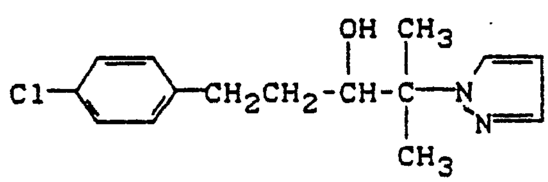

27,4 g (0.1 Mol) 5-(4-Chlorphenyl)-2-methyl-2-(pyrazol-1-yl)-pent-3-on-4-en werden in 200 ml Methanol mit 7 g Raney-Nickel versetzt und 4 Stunden bei 60 °C mit 40-50 bar Wasserstoffdruck hydriert. Nach Absaugen des Katalysators und Abdestillieren des Methanols werden 27,2 g 5-(4-Chlorphenyl)-2-methyl-2-(pyrazol-1-yl)-pentan-3-ol vom Brechungsindex $n_D^{20}$ = 1,5438 erhalten.

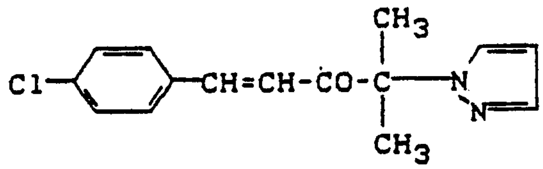

(IV-2)

69,5 g (0,45 Mol) 2-Methyl-2-(pyrazol-1-yl)-butan-3-on werden in 200 ml Ethanol gelöst, es werden 44 ml Wasser zugegeben sowie 64 g (0,45 Mol) p-Chlorbenzaldehyd in 50 ml Ethanol. Dann tropft man 1,35 g Natriumhydroxid in 13,5 ml Wasser zu. Die Reaktion ist exotherm bis 30 °C. Nach einer Stunde Nachrühren werden weitere 0,44 g (0,011 Mol) Natriumhydroxid in 4,4 ml Wasser zugetropft und nach 3 Tagen Stehen bei Raumtemperatur das Lösungsmittel abdestilliert, der Rückstand in 400 ml Methylenchlorid aufgenommen und mit 300 ml Wasser und 10 ml Eisessig je 3 mal gewaschen. Die organische Phase wird im Wasserstrahlvakuum vom Lösungsmittel abdestilliert und aus Petrolether umkristallisiert.

Man erhält 91,6 g (74,2 % der Theorie) 5-(4-Chlorphenyl)-2-methyl-(pyrazol-1-yl)-pent-3-on-4-en vom Schmelzpunkt 60 °C.

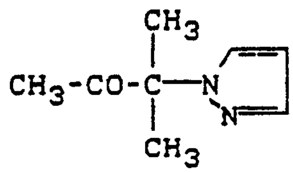

(XI-1)

Zu einer Schmelze von 4 Mol Pyrazol werden 1 Mol Methylbromisopropyl-keton bei 100 °C zugetropft und 1 Stunde nachgerührt. Nach üblicher Aufarbeitung erhält man 2-Methyl-2-(pyrazol-1-yl)-butan-3-on in einer Ausbeute von 70 %.

## Beispiel 2

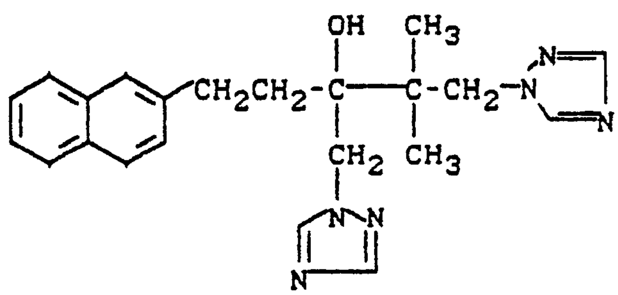

(I-2)

Eine Lösung von 10 g (0,031 Mol) 2-[1,1-Dimethyl-2-(1,2,4-triazol-1-yl)]-ethyl-2-(naphth-2-yl-ethyl)-oxiran, 2,4 g (0,0348 Mol) 1,2,4-Triazol, 0,4 g Natriumhydroxid, 0,1 g Wasser und einer Spatelspitze α,α'-Azoisobutyronitril in 100 ml Dimethylformamid wird 5 Stunden bei 100 °C gerührt, anschließend abgekühlt und eingeengt. Der Rückstand wird in Dichlormethan gelöst, dreimal mit Wasser gewaschen, über Natriumsulfat getrocknet und eingeengt. Der Rückstand wird säulenchromatographisch (Kieselgel; Essigester und anschließend Essigester/Methanol = 1:1) gereinigt.

Man erhält 6,0 g (49,6 % der Theorie) 4,4-Dimethyl-1-(2-naphthyl)-5-(1,2,4-triazol-1-yl)-3-(1,2,4-triazol-1-yl-methyl)-3-pentanol vom Schmelzpunkt 77-80 °C.

## Herstellung von Ausgangsprodukten

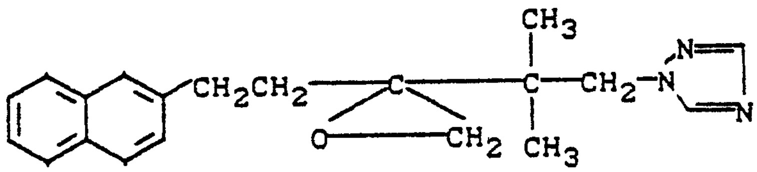

(II-2)

(Variante a)

Eine Lösung von 22 g (0,0721 Mol) 2-[1,1-Dimethyl-2-(1,2,4-triazol-1-yl)]-ethyl-4-(2-naphthyl)-1-buten in 50 ml Dichlormethan wird unter Rückfluß erhitzt und innerhalb von 1 1/2 Stunden eine Lösung von 27,9 g (0,129 Mol) 80%-ige m-Chlorperbenzoesäure in 270 ml Dichlormethan eingetropft. Anschließend wird weitere 4 Stunden unter Rückfluß erhitzt, dann abgekühlt, dreimal mit 1-normaler Natronlauge und zweimal mit Wasser gewaschen, die organische Phase über Natriumsulfat getrocknet und eingeengt. Es werden 19,0 g (82,1 % der Theorie) 2-[1,1-Dimethyl-2-(1,2,4-triazol-1-yl)]-ethyl-2-(naphth-2-yl-ethyl)-oxiran als schwarzes Öl erhalten. $^1$H-NMR (in $CDCl_3$; 300 MHz) $\delta$ = 2.60 (1H, d) und 2,73 (1H, d) für die Epoxid-Methylengruppe.

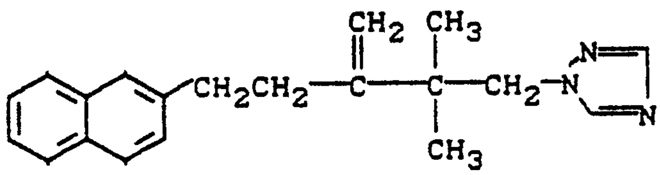

(VI-1)

Eine Suspension von 57,3 g (0,16 Mol) Methyltriphenylphosphoniumbromid und 18,4 g (0,16 Mol) Kalium-tertbutylat in 250 ml absolutem Toluol wird unter trockenem Stickstoff bei 90 °C erwärmt. Über 30 Minuten wird eine Lösung von 36,8 g (0,12 Mol) 4,4-Dimethyl-1-(2-naphthyl)-5-(1,2,4-triazol-1-yl)-3-pentanon in 200 ml absolutem Toluol eingetropft, das Reaktionsgemisch anschließend 2 Stunden bei 90 °C nachgerührt, abgekühlt, zweimal mit Wasser gewaschen und eingeengt. Der Rückstand wird in Essigester aufgenommen, auf 5 °C gekühlt und der dabei gebildete Kristallbrei (Triphenylphosphoniumoxid) abfiltriert. Das Filtrat wird eingeengt und der Rückstand mittels Säulenchromatographie gereinigt (Kieselgel, Dichlormethan/Essigester = 9 : 1).
Man erhält 27,0 g (73,8 % der Theorie) 2-[1,1-Dimethyl-2-(1,2,4-triazol-1-yl)-ethyl-4-(2-naphthyl)-1-buten als gelbes Öl.
$^1$H-NMR (in $CDCl_3$; 300 MHz): $\delta$ = 4.91 (1H, s) und 5.05 (1H, s) für die olefinische Methylengruppe.

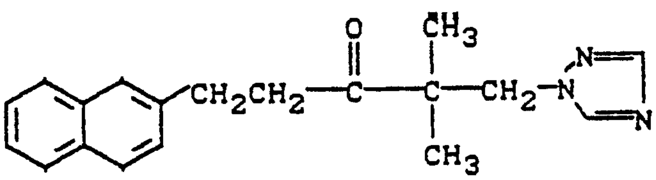

(IV-3)

Eine Lösung von 67 g (0,22 Mol) 4,4-Dimethyl-1-(2-naphthyl)-5-(1,2,4-triazol-1-yl)-1-penten-3-on in 400 ml Methanol wird mit 15 g Raney-Nickel versetzt und im Autoklaven 4 Stunden bei 90 °C unter 90 bar Wasserstoff gerührt. Nach Filtrieren und Einengen erhält man 55 g (81,4 % der Theorie) 4,4-Dimethyl-1-(2-naphthyl)-5-(1,2,4-triazol-1-yl)-3-pentanon als gelben Feststoff vom Schmelzpunkt 134 °C.

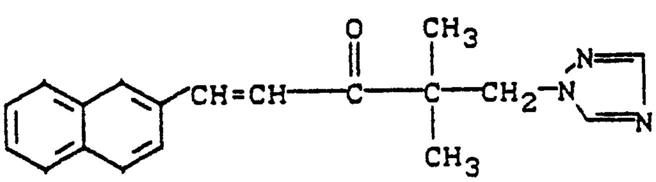

(IV-4)

59,3 g (0.38 Mol) 2-Naphthaldehyd und 63,5 g (0,38 Mol) 3,3-Dimethyl-4-(1,2,4-triazol-1-yl)-2-butanon

werden in 200 ml Ethanol gelöst und mit einer Lösung von 1,5 g Natriumhydroxid in 15 ml Wasser versetzt. Das Gemisch wird 1 Stunde bei Raumtemperatur gerührt, mit 0,5 g festem Natriumhydroxid versetzt, 1 Stunde weitergerührt, mit 50 ml Wasser versetzt und über Nacht gerührt. Das Reaktionsgemisch wird mit 100 ml Wasser versetzt, der Niederschlag abgesaugt, in Dichlormethan gelöst, zweimal mit Wasser gewaschen, über Natriumsulfat getrocknet und eingeengt.

Man erhält 115 g (99 % der Theorie) 4,4-Dimethyl-1-(2-naphthyl)-5-(1,2,4-triazol-1-yl)-1-penten-3-on als weißen Feststoff vom Schmelzpunkt 103-104 °C.

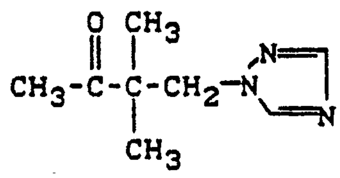

(XI-2)

In Lösung von 138 g (2 Mol) 1,2,4-Triazol in 1600 ml absolutem Dimethylformamid werden 60 g (2 Mol) Natriumhydrid (80 %-ig in Paraffinöl) über 3 Stunden eingetragen, wobei Wasserstoff kräftig entweicht. Das Gemisch wird 30 Minuten bei Raumtemperatur nachgerührt. 388 g (2 Mol) 3,3-Dimethyl-4-[(methylsulfonyl)-oxy]-2-butanon werden über 15 Minuten eingetropft und das ganze dann bei 95 °C über Nacht gerührt. Das Reaktionsgemisch wird vorsichtig mit 100 ml Wasser versetzt, in 4 l Wasser gegossen und sechsmal mit Dichlormethan extrahiert. Die vereinigten organischen Phasen werden einmal mit viel Wasser gewaschen, über Natriumsulfat getrocknet und eingeengt (unter Hochvakuum). Nach Säulenchromatographie (Kieselgel, Dichlormethan/Essigester = 1:1) erhält man 290 g (86,8 % der Theorie) 3,3-Dimethyl-4-(1,2,4-triazol-1-yl)-2-butanon als dunkles Öl mit einem Gehalt von 97 % nach GC.

$^1$H-NMR (in $CDCl_3$; 300 MHz) : $\delta$ = 1.23 (6H, s; $C(CH_3)_2$), 2.19 (3H, s; $CH_3CO$), 4.32 (2H, s; $CH_2$-Triazol), 7.90 (1H, s) und 8.12 (1H, s) für die Triazol-Ringprotonen.

Entsprechend den Herstellungsbeispielen 1 und 2 sowie den erfindungsgemäßen Verfahrensangaben werden die nachfolgenden Verbindungen der allgemeinen Formel (I) erhalten:

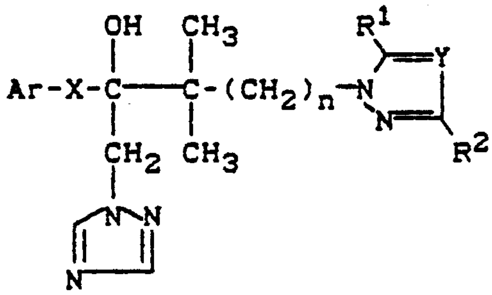

(I)

| Bsp. Nr. | Ar | X | n | 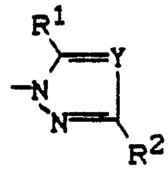 | Fp(°C) |
|---|---|---|---|---|---|
| 3 | 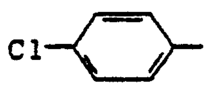 | $-CH_2CH_2-$ | 1 | 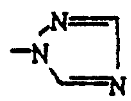 | Harz |
| 4 | 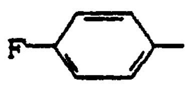 | $-CH_2CH_2-$ | 1 | 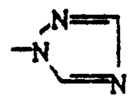 | zähes Öl |
| 5 | 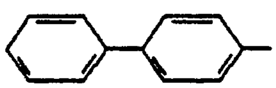 | $-CH_2CH_2-$ | 1 | 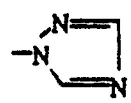 | 96-98 |
| 6 | 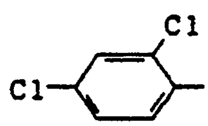 | $-CH_2CH_2-$ | 1 | 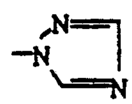 | 116-117 |
| 7 | 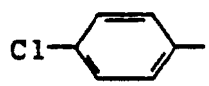 | -CH=CH- | 0 | 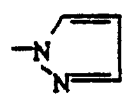 | 132 (trans-Isomeres) |
| 8 | 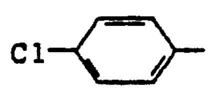 | -CH=CH- | 0 | 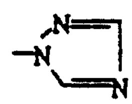 | 198 |

| Bsp. Nr. | Ar | X | n | 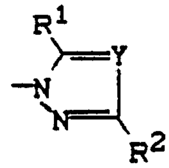 | Fp(°C) |
|---|---|---|---|---|---|
| 9 | 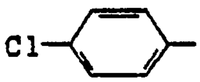 | $-SCH_2-$ | 0 | 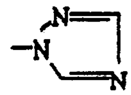 | 92 |
| 10 | 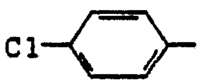 | $-OCH_2-$ | 0 | 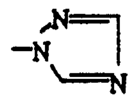 | 95 |
| 11 | 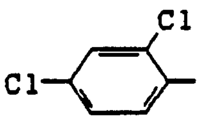 | $-OCH_2-$ | 0 | 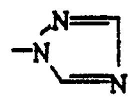 | 140 |
| 12 | 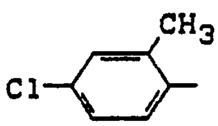 | $-OCH_2-$ | 0 | 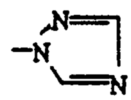 | 154 |
| 13 | 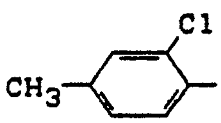 | $-OCH_2-$ | 0 | 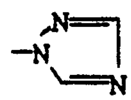 | 129 |
| 14 | 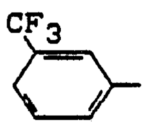 | $-OCH_2-$ | 0 | 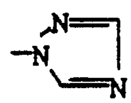 | 122 |
| 15 | 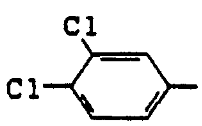 | $-OCH_2-$ | 0 | 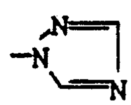 | 150 |
| 16 | 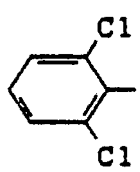 | $-CH_2CH_2-$ | 1 | 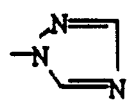 | 82-86 |

| Bsp. Nr. | Ar | X | n | 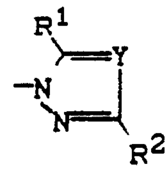 | Fp(°C) |
|---|---|---|---|---|---|
| 17 | 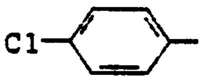 | $-CH_2CH_2-$ | 0 | 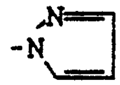 | -50 (x HCl) (hygroskopisch) |
| 18 | 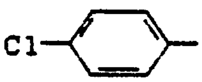 | $-CH_2CH_2-$ | 0 | 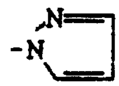 | 110 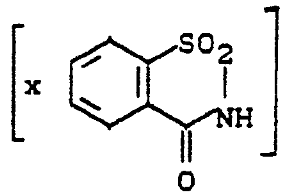 |
| 19 | 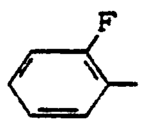 | $-CH_2CH_2-$ | 0 | 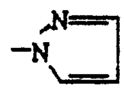 | -35 |
| 20 | 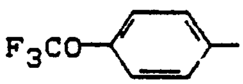 | $-CH_2CH_2-$ | 0 | 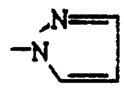 | 92 |
| 21 | 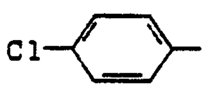 | $-CH_2CH_2-$ | 0 | 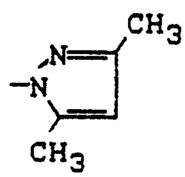 | Öl |
| 22 | 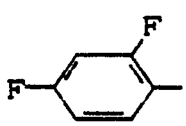 | $-CH_2CH_2-$ | 0 | 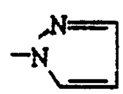 | 104 |
| 23 | 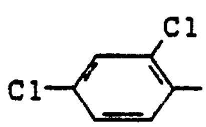 | $-CH_2CH_2-$ | 0 | 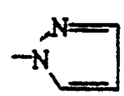 | 84 |
| 24 | 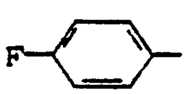 | $-CH_2CH_2-$ | 0 | 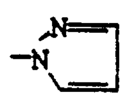 | 120 |

| Bsp. Nr. | Ar | X | n | 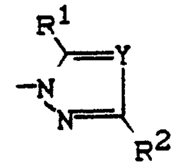 | Fp(°C) |
|---|---|---|---|---|---|
| 25 | 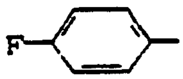 | $-CH_2CH_2-$ | 0 | 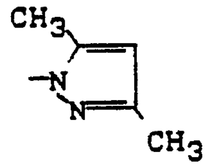 | $n_D^{20}$ =1.5394 |
| 26 | 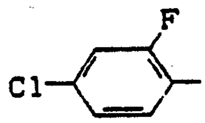 | $-CH_2CH_2-$ | 0 | 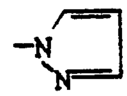 | 102 |
| 27 | 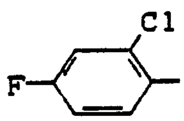 | $-CH_2CH_2-$ | 0 | 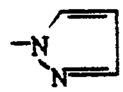 | $n_D^{20}$ =1.5399 |
| 28 | 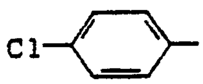 | $-OCH_2-$ | 0 | 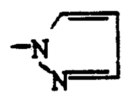 | 146 |
| 29 | 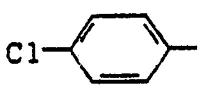 | $-CH_2CH_2-$ | 0 | 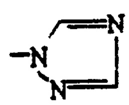 | zähfl. Öl |
| 30 | 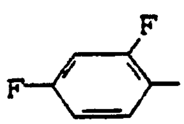 | $-CH_2CH_2-$ | 1 | 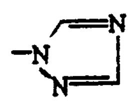 | 109 |

<u>Verwendungsbeispiele</u>

In dem nachfolgenden in-vitro-Beispiel werden die nachstehend angegebenen Verbindungen als Vergleichssubstanzen eingesetzt:

(A)

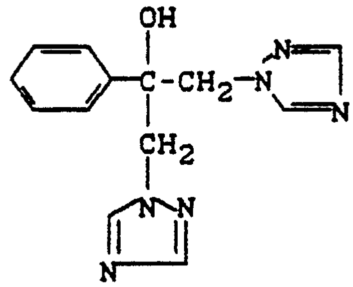

(bekannt aus EP 0 044 605)

(B)

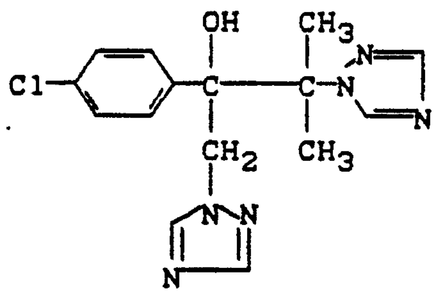

(bekannt aus EP 0 120 276)

## Verwendungsbeispiele

### Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nährmedium dienten

a) für Dermatophyten und Schimmelpilze:
Sabouraud's milieu d'epreuve

b) für Hefen:
Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test Zeigen die erfindungsgemäßen Verbindungen der Herstellungsbeispiele 1, 2, 5, 7, 9, 10, 11, 12 und 13 ein gutes antimykotisches Wirkungsspektrum.

Tabelle A

Antimykotische in-vitro-Wirksamkeit
MHK-Werte in µg/ml Nährmedium bei

| Wirkstoff | Tricho-phyton mentagr. | Micro-sporum canis | Candida albi-cans | Torulop-sis glabrata | Asper-gillus fumi-gatus |
|---|---|---|---|---|---|
| (A)(bekannt) | 64 | - | 64 | >64 | >64 |
| (B)(bekannt) | 8 | >64 | >64 | >64 | >64 |
| Verbindungen gemäß Her-stellungs-beispiel | | | | | |
| 1 | <1 | 2 | 2 | 2 | 2 |
| 2 | 4 | - | 32 | >64 | >64 |
| 5 | 2 | - | 8 | 32 | 8 |
| 7 | <1 | 4 | <1 | 16 | 4 |
| 9 | 2 | - | <1 | 32 | 64 |
| 10 | 8 | - | 2 | >64 | >64 |
| 11 | 2 | - | 8 | >64 | >64 |
| 12 | 4 | - | 16 | >64 | >64 |
| 13 | 2 | - | 32 | >64 | >64 |
| 17 | <1 | 2 | 16 | 32 | 2 |
| 18 | <1 | 4 | 8 | 2 | 2 |

**Tabelle A** (Fortsetzung)

| Wirkstoff | Trichophyton mentagr. | Microsporum canis | Candida albicans | Torulopsis glabrata | Aspergillus fumigatus |
|---|---|---|---|---|---|
| 19 | 2 | - | 16 | 8 | 16 |
| 20 | <1 | 2 | 4 | 4 | <1 |
| 21 | <1 | <1 | 8 | 16 | 2 |
| 22 | 2 | - | 2 | 8 | 16 |
| 23 | 2 | - | 8 | 2 | 8 |
| 24 | <1 | 4 | <1 | 4 | 8 |
| 25 | 2 | - | 16 | >64 | 4 |
| 26 | <1 | 2 | 8 | 32 | 2 |
| 27 | 4 | - | 8 | 16 | 32 |
| 28 | <1 | 8 | 32 | 16 | 16 |
| 29 | <1 | 4 | <1 | 32 | 16 |

Beispiel B

Antimykotische in-vivo-Wirksamkeit (oral) bei Mäusecandidose

Versuchsbeschreibung :

Mäuse vom Typ SPF-$CF_1$ wurden intravenös mit 1 - 2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 10 - 100 mg/kg Körpergewicht der Präparate oral behandelt.

Ergebnis:

Unbehandelte Tiere starben 3 bis 6 Tage post infektionem. Die Überlebensrate am 6.Tag post infektionem betrug bei unbehandelten Kontrolltieren etwa 5 %.

In diesem Test zeigen z.B. die erfindungsgemäßen Verbindungen 5, 8 und 10 eine gute antimykotische Wirkung.

Tabelle B

| Antimykotische in-vivo-Wirksamkeit (oral) bei Mäusecandidose | |
|---|---|
| Verbindungen gemäß Herstellungs-Beispielen | |
| 5 | + + + |
| 8 | + + + + |
| 10 | + + + + + |
| Zeichenerklärung: | |
| + + + + + = sehr gute Wirkung = 90 % Überlebende am 6.Tag.p.i. | |
| + + + + = gute Wirkung = 80 % Überlebende am 6.Tag.p.i. | |
| + + + = Wirkung = 60 % Überlebende am 6.Tag.p.i. | |
| + + = schwache Wirkung = 40 % Überlebende am 6.Tag.p.i. | |
| + = Spur Wirkung = unter 40 % Überlebende am 6. Tag p.i. | |
| k.W. = kein Unterschied zur unbehandelten Infektionskontrolle | |

Beispiel C / Formulierungen

| 1.) Lösung: | |
|---|---|
| Wirkstoff gemäß Formel (I): | 10 g |
| Alkohol, rein (96 %ig) : | 300 g |
| Isopropylmyristat : | 526 g |
| | 836 g |

| 2.) Creme: | |
|---|---|
| Wirkstoff gemäß Formel (I): | 10 g |
| Aralcel 60 : (Sorbitan-monostearat) | 20 g |
| Tween 60 : (Polyoxyethylen (2) -sorbitanmonostearat) | 15 g |
| Walrat, künstlich : (Mischung vom Estern von gesättigten Fettsäuren $C_{14}$-$C_{18}$ und Fettalkoholen $C_{14}$-$C_{18}$) | 30 g |
| Lanette O : | 100 g |
| Eutanol G : (2-Octyl-dodecanol) | 135 g |
| Benzylalkohol : | 10 g |
| Wasser, entmineralisiert : | 680 g |
| | 1000 g |

## Ansprüche

1. Bisazolyl-Derivate der allgemeinen Formel,

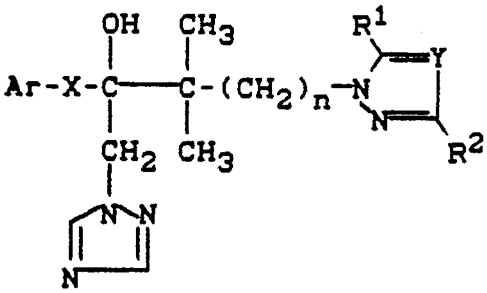

in welcher
Ar für gegebenenfalls substituiertes Aryl steht,
X für eine Gruppierung $-CH_2CH_2-$, $-CH=CH-$, $-OCH_2-$oder, $-SCH_2-$ steht, wobei das Sauerstoff- oder Schwefelatom mit dem Arylrest direkt verbunden ist,
Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht,
sowie deren pharmakologisch verträgliche Säureadditions-Salze zur Bekämpfung von Krankheiten.

2. Bisazolyl-Derivate gemäß Anspruch 1, in denen
Ar für gegebenenfalls einfach bis dreifach, gleich oder verschieden substituiertes Phenyl steht, wobei als Substituenten genannt seien: Halogen, Alkyl mit 1 bis 4 Kohlenstoffatomen, Alkoxy und Alkylthio mit jeweils 1 bis 2 Kohlenstoffatomen, Nitro, Halogenalkyl, Halogenalkoxy und Halogenalkylthio mit jeweils 1 bis 2 Kohlenstoffatomen und 1 bis 5 gleichen oder verschiedenen Halogenatomen, wie vorzugsweise Fluor- und Chloratomen, Alkoximinoalkyl mit 1 bis 4 Kohlenstoffatomen im Alkoxyteil und 1 bis 2 Kohlenstoffatomen im Alkylteil sowie jeweils gegebenenfalls durch Halogen und Alkyl mit 1 bis 2 Kohlenstoffatomen substituiertes Phenyl, Phenoxy, Benzyl und Benzyloxy;
Ar außerdem für gegebenenfalls einfach bis dreifach, gleich oder verschieden substituiertes Naphthyl steht, wobei als Substituenten genannt seien: Halogen, Alkyl und Alkoxy mit jeweils 1 bis 2 Kohlenstoffatomen sowie Halogenalkoxy mit 1 bis 2 Kohlenstoffatomen und 1 bis 5 gleichen oder verschiedenen Halogenatomen, wie vorzugsweise Fluor-und Chloratomen;
X für die Gruppierungen $-CH_2CH_2-$, $-OCH_2-$, $-SCH_2-$oder $-CH=CH-$ steht;
Y für ein Stickstoffatom oder die Gruppierung $CR^3$ steht;
$R^1$, $R^2$ und $R^3$ für Wasserstoff, Chlor, Brom oder Methyl stehen und
n für die Zahlen 0, 1 oder 2 steht sowie deren pharmakologisch verträgliche Säureadditions-Salze zur Bekämpfung von Krankheiten.

3. Bisazolyl-Derivate gemäß Anspruch 1, in denen
Ar für gegebenenfalls einfach bis dreifach, gleich oder verschieden substituiertes Phenyl steht, wobei als Substituenten genannt seien: Fluor, Chlor, Brom, Methyl, Methoxy, Methylthio, Nitro, Trifluormethyl, Trifluormethoxy, Trifluormethylthio, Methoximinomethyl, Methoximinoethyl, Ethoximinomethyl sowie jeweils gegebenenfalls durch Fluor, Chlor und Methyl substituiertes Phenyl, Phenoxy, Benzyl und Benzyloxy; oder Ar für Naphthyl steht;
X für die Gruppierungen $-CH_2CH_2-$, $-OCH_2-$, $-SCH_2-$oder $-CH=CH-$ steht;
Y für ein Stickstoffatom oder die Gruppierung $CR^3$ steht;
$R^1$, $R^2$ und $R^3$ für Wasserstoff, Chlor oder Methyl stehen und
n für die Zahlen 0, 1 oder 2 steht sowie deren pharmakologisch verträgliche Säureadditions-Salze zur Bekämpfung von Krankheiten.

4. Bisazolyl-Derivate gemäß Ansprüchen 1 - 3 zur Bekämpfung von Mykosen.

5. Arzneimittel, enthaltend Bisazolyl-Derivate gemäß Ansprüchen 1 - 3.

6. Antimykotische Mittel enthaltend Bisazolyl-Derivate gemäß Ansprüchen 1 - 3.

7. Verwendung der Bisazolyl-Derivate gemäß Ansprüchen 1 - 3 bei der Bekämpfung von Krankheiten.

8. Verwendung der Bisazolyl-Derivate gemäß Ansprüchen 1 - 3 bei der Bekämpfung von Mykosen.